# EUROPÄISCHE PATENTSCHRIFT

(11) **EP 2 957 233 B1**
(45) Veröffentlichungstag und Bekanntmachung des Hinweises auf die Patenterteilung: **24.10.2018**
(21) Anmeldenummer: 15169465.0
(22) Anmeldetag: 27.05.2011
(51) Int. Cl.: A61B 10/02, A61B 17/29, A61B 17/3205, A61B 17/00

(54) **GELENK-BIOPSIENADEL ZUR ENTNAHME VON GEWEBEPROBEN**
JOINT BIOPSY NEEDLE FOR TAKING TISSUE SAMPLES
AIGUILLE À BIOPSIE ARTICULÉE DESTINÉE AU PRÉLÈVEMENT D'ÉCHANTILLONS DE TISSUS

(30) Priorität: 01.06.2010 DE 102010017185
(43) Veröffentlichungstag der Anmeldung: 23.12.2015
(62) Teilanmeldung aus: 11722332.1
(73) Patentinhaber: Hipp Medical AG, 78600 Kolbingen (DE)
(72) Erfinder: Hipp, Markus, 78600 Kolbingen (DE)
(74) Vertreter: Kuhnen & Wacker Patent- und Rechtsanwaltsbüro PartG mbB

(56) Entgegenhaltungen:
- WO-A1-01/89388
- WO-A2-2004/075719
- DE-A1- 10 059 264
- US-A- 5 911 701
- US-A1- 2008 103 504
- US-B1- 6 419 641

## Beschreibung

Die vorliegende Erfindung betrifft eine Gelenk-Biopsienadel zur Entnahme von Gewebeproben nach dem Oberbegriff des Anspruchs 1.

Derartige Gelenk-Biopsienadeln weisen üblicherweise eine Trokarspitze und eine Führungshülse auf, die an eine Grundfläche der Trokarspitze angrenzt, und in einem der Trokarspitze benachbarten Bereich eine seitliche Ausnehmung aufweist. Ferner weisen solche Biopsienadeln häufig einen in Längsrichtung der Führungshülse darin verschiebbaren Stempel auf, der auf einer auf die Trokarspitze zuweisenden Seite eine Schneide aufweist, wobei das im Bereich der seitlichen Ausnehmung der Führungshülse zu entnehmende Gewebe, das durch die seitliche Ausnehmung in die Führungshülse gelangen kann, durch die Schneide abtrennbar ist. Die Führungshülse ist dabei in einem Handteil festgelegt.

Im Falle von z.B. Rheuma-Patienten treten Entzündungen eines Gelenks auf der Gelenkhaut auf. Dabei kommt es häufig zu Schwellungen und zur Ausbreitung von Bakterien in umliegendes Gewebe. Um in solchen Fällen ein geeignetes Medikament zu verabreichen, ist es notwendig festzustellen, um welches Bakterium es sich im Gewebe handelt.

Um dies zu realisieren, muß eine Gewebeprobe entnommen werden, die später in einem Labor untersucht werden kann. Herkömmliche, aus dem Stand der Technik bekannte Gewebeentnahmevorrichtungen sind beispielsweise Hohlbiopsienadeln, mit welchen unter Verwendung verschiedener Mechanismen Gewebeproben menschlicher, tierischer, pflanzlicher und/oder technischer Strukturen entnommen werden können.

Im Rahmen derartiger Gewebeentnahmen werden zum Beispiel seitliche und koaxiale Schneidemechanismen in Kombination mit Schraubmechanismen oder nach dem Korkenzieherprinzip, insbesondere bei der sogenannten Nadelbiopsie, angewandt. Des Weiteren findet auch die vakuumassistierte Biopsie unter Zuhilfenahme eines Vakuum erzeugenden Spritzen-/Kolbenmechanismus oder eines externen Vakuumgeräts Anwendung, wobei zu entnehmendes Gewebe durch den in der Biopsienadel erzeugten Unterdruck aus dem zu untersuchenden Gewebeverbund in die Biopsienadel, beispielsweise durch ein seitliches Fenster eines proximalen Hohlnadelbereichs, gesaugt wird. Hierbei kann das in den Hohlnadelbereich gesaugte Gewebe durch den Unterdruck abgerissen, oder durch ein Schneidewerkzeug abgetrennt werden.

Die eine Vakuum-Biopsievorrichtung diskutierende, internationale Patentanmeldung PCT/DE03/00844, veröffentlicht als WO 03/077767 A1 offenbart zum Beispiel eine Biopsievorrichtung zur Entnahme von Gewebeproben, die aus einem Handstück besteht, in das eine hohle Biopsienadel eingelegt wird, wobei ein Teil des über das Handstück hinausragenden Teils der geraden Biopsienadel mit seinem Probeentnahmeraum in das zu untersuchende Gewebe eingebracht wird, und das Gewebe mittels Vakuum in den Probeentnahmeraum eingesaugt, anschließend mittels einer Probeabtrenneinrichtung abgetrennt, und abschließend entnommen wird.

Diese Vorrichtungen und Verfahren sind somit gut geeignet, um aus einem leicht zugänglichen Gewebe eine Probe zu entnehmen. Wenn jedoch ein Probeentnahmebereich von einem anderen Körperteil und/oder Gewebebereich, welcher nicht verletzt oder berührt werden darf, verdeckt wird, ist es mit den obenstehenden Vorrichtungen nur schwierig oder gar nicht möglich, eine entsprechende Gewebeprobe zu entnehmen.

Aus der DE 100 59 264 A1 ist ein zur Gewebeentnahme bestimmtes endoskopisches Instrument bekannt, das eine Aussenhülle, die am Innenumfang ihres distalen Endes eine ringförmige Schneide und in einem Bereich nahe der Schneide eine laterale Ausnehmung hat, aufweist. In der Aussenhülle ist eine innere Nadel entlang der Längsachse vor- und zurückbewegbar. Die innere Nadel hat an ihrem distalen Ende eine Schneide zum Abschneiden von in die Ausnehmung gelangtem Gewebe sowie eine nach vorne weisende Nadelspitze. Die innere Nadel kann von dem Benutzer zwischen zwei Stellungen vor- und zurückbewegt werden, wobei das distale Ende der inneren Nadel in der einen Stellung so in die Aussenhülle eingezogen ist, dass es von der Ausnehmung entfernt ist und in der anderen Stellung aus dem distalen Ende der Aussenhülle heraussteht.

Ausgehend vom Stand der Technik ist es Aufgabe der vorliegenden Erfindung, eine auch an ungünstigen Probeentnahmestellen einfach zu bedienende Gelenk-Biopsienadel zur Entnahme von Gewebeproben bereitzustellen.

Diese Aufgabe wird erfindungsgemäß durch eine Gelenk-Biopsienadel mit den Merkmalen des Anspruches 1 gelöst.

Erfindungsgemäß wird hierbei eine Gelenk-Biopsienadel zur Entnahme von Gewebeproben mit einer Trokarspitze und einer im Wesentlichen steifen Führungshülse, die an eine Grundfläche der Trokarspitze angrenzt, und in einem der Trokarspitze benachbarten Bereich eine seitliche Ausnehmung aufweist, vorgesehen, wobei die Biopsienadel einen in Längsrichtung der Führungshülse darin verschiebbaren Stempel umfaßt, der auf einer auf die Trokarspitze zuweisenden Seite eine Schneide aufweist, wobei das im Bereich der seitlichen Ausnehmung der Führungshülse zu entnehmende Gewebe, das durch die seitliche Ausnehmung in die Führungshülse gelangen kann, durch die Schneide abtrennbar ist.

Dabei ist die Führungshülse in ein Handteil eingebracht, wobei die Führungshülse erfindungsgemäß an wenigstens einer Stelle zwischen der Trokarspitze und dem Handteil einen gekrümmten Verlauf aufweist, und der Stempel über ein in der Führungshülse geführtes, flexibles Kraftübertragungselement verschiebbar ist.

Hierbei ist insbesondere die kleine, einfache, und durch die gekrümmte Führungshülse dennoch äußerst effektive Bau- und Funktionsweise der Gelenk-Biopsienadel von Vorteil.

Der gekrümmte Verlauf der Führungshülse erleichtert es der behandelnden Person, die Biopsienadel in einem gewünschten Gewebeentnahmebereich des Körpers zu positionieren. Dabei ist das Handteil auch bei einem schrägen Einbringen nicht hinderlich, da es nicht auf dem Körper zu liegen kommt, was einen eingeschränkten Bewegungsfreiraum zur Folge hätte.

Darüber hinaus sind durch den gekrümmten Verlauf der Führungshülse Gewebebereiche zugänglich, welche mit einem geraden Verlauf der Führungshülse bzw. der Biopsienadel nicht, oder nur sehr schwierig erreichbar wären. Zum Beispiel beim Vordringen mit der Biopsienadel in einen Gewebebereich, welcher durch ein Körperteil, welches nicht verletzt oder berührt werden darf, verdeckt ist.

Dabei kann das flexible Kraftübertragungselement einem beliebigen Verlauf der Führungshülse folgen, und somit stets die erforderliche Kraft auf den Schneidestempel übertragen, um diesen entsprechend in der Führungshülse zu bewegen. Da die behandelnde Person hierbei weder auf zusätzliche Ausrüstungselemente, noch auf eine etwaig nötige Leistungsquelle angewiesen ist, kann die Gewebeentnahme schnell und einfach vorbereitet bzw. durchgeführt werden.

Aufgrund der einfach gestalteten Bauelemente der erfindungsgemäßen Biopsienadel ist diese im Vergleich zu Vakuumbiopsienadeln und/oder Biopsienadeln mit zusätzlicher elektrischer Ausrüstung eine kostengünstigere Lösung.

Auf das flexible Kraftübertragungselement ist über eine Betätigungsvorrichtung und einen in dem Handteil geführten Kraftübertragungskolben eine Kraft übertragbar, wobei sich an einem dem Kraftübertragungskolben zugewandten Ende des Stempels eine erste Aufnahme, und an einem dem Stempel zugewandten Ende des Kraftübertragungskolbens eine zweite Aufnahme für das Kraftübertragungselement befinden. Über das Betätigungselement kann die Biopsienadel von der behandelnden Person ohne aufwändige zusätzliche Ausrüstung einfach und schnell bedient werden. Darüber hinaus ermöglichen der Zusammenbau von Kraftübertragungskolben, Stempel und Kraftübertragungselement über die erste und zweite Aufnahme eine einfache und schnelle Montage bzw. Demontage.

Überdies sind ein distaler Schaft des Kraftübertragungskolbens und ein zugeordnetes Führungsloch im Handteil, in welchem der Kraftübertragungskolben geführt wird, verdrehsicher, insbesondere jeweils exzentrisch und komplementär zueinander, ausgebildet. Somit kann der Schaft gegenüber dem Handteil nicht mehr verdreht werden, wodurch die radiale Position immer dieselbe bleibt, und ein Lösen des Kraftübertragungselements von der ersten und der zweiten Aufnahme aufgrund einer Torsionskraft verhindert werden kann.

Das flexible Kraftübertragungselement kann zum Beispiel als Kunststoffschlauch ausgebildet sein, welcher schnell und einfach bereitstellbar ist, und somit eine kostengünstige Lösung darstellt. Ein solcher Hohlkörper kann zudem als Durchgangspassage für ein in den zu untersuchenden Bereich zu injizierendes Mittel dienen.

Der Stempel kann im Inneren einen zur Schneide hin geöffneten Hohlraum aufweisen, in welchen das zu sammelnde Gewebe aufnehmbar ist. In diesem Hohlraum kann eine entnommene Gewebeprobe so lange aufgenommen sein, bis die Biopsienadel aus dem Körper entfernt worden ist, um sie anschließend daraus zu entnehmen. Der Hohlraum kann dabei durch Formschluss mit einer Grundfläche der Trokarspitze derart abgekapselt werden, daß kein ggf. mit Bakterien oder anderen Verunreinigungen versetztes Gewebe beim Herausnehmen der Biopsienadel aus dem Körper mit anderen Gewebebereichen bzw. Körperteilen in Berührung kommt.

Ferner kann eine Trokarspitze in Form einer mehrseitigen Schneidespitze mit konkaven Schneidekanten ausgebildet sein, wodurch eine Schnittkraft linear distal besser übertragen und besser in den zu untersuchenden Bereich vorgedrungen werden kann.

Die Trokarspitze kann außerdem auf mindestens einer Seite wenigstens eine Bohrung aufweisen, wodurch ein Durchfluß einer Körperflüssigkeit durch die Trokarspitze erlaubt wird. Somit kann das Risiko, daß während der Untersuchung bestimmte Flüssigkeitskreisläufe des Körpers durch die Trokarspitze blockiert werden, auf ein Minimum beschränkt werden.

An dem der Trokarspitze entgegengesetzten Ende der Gelenk-Biopsienadel, am distalen Ende des Handteils, kann eine Anschlusseinrichtung für eine Druckerzeugungsvorrichtung vorgesehen sein, mittels der ein Medikament, während sich die Biopsienadel in dem zu untersuchenden Bereich befindet, über eine durchgehende Kanülierung durch das Handteil, das Kraftübertragungselement, den Stempel, und/oder die Trokarspitze in den zu behandelnden Bereich injizierbar ist. Dadurch kann die Biopsienadel nicht nur als Gewebeprobeentnahmevorrichtung dienen, sondern auch als Medikament-Injiziereinrichtung. Ein vorsorgliches oder anschließendes zweites Einstechen in den Körper zum Einbringen des Medikaments kann somit vermieden werden.

Im Handteil kann außerdem ein Rückstell-Federelement für den Stempel angeordnet sein, welches den Schneidestempel nach Beendigung des Schneidevorgangs über das Kraftübertragungselement und den Kraftübertragungskolben in seine Grundposition zurückführt. Dadurch kann die Biopsienadel einfach mit einer Hand bedient werden. Das Rückstell-Federelement ist dabei bevorzugt so im Handteil untergebracht, daß es weder von innen noch von außen mit Gewebe oder anderen Substanzen in Berührung kommen kann, wodurch eine besonders lange und zuverlässige Funktion gewährleistet werden kann.

Die Erfindung wird im Folgenden anhand der in den beigefügten Figuren dargestellten Ausführungsformen näher beschrieben.

Es zeigt:
- Fig. 1: eine Schnittansicht einer erfindungsgemäßen Gelenk-Biopsienadel bzw. Biopsienadel;
- Fig. 2: eine Seitenansicht der erfindungsgemäßen Biopsienadel;
- Fig. 3: eine Schnittansicht einer Trokarspitze der erfindungsgemäßen Biopsienadel;
- Fig. 4: eine Schnittansicht eines Kraftübertragungsstempels der erfindungsgemäßen Biopsienadel;
- Fig. 5a: eine Draufsicht auf ein Handteil der erfindungsgemäßen Biopsienadel;
- Fig. 5b: eine Seitenansicht eines Handteils der erfindungsgemäßen Biopsienadel;
- Fig. 5c: eine Schnittansicht eines Handteils der erfindungsgemäßen Biopsienadel;
- Fig. 6: eine perspektivische Ansicht einer alternativen Ausführungsform der Biopsienadel;
- Fig. 7: eine vergrößerte Ansicht der Trokarspitze gemäß der alternativen Ausführungsform der Biopsienadel;
- Fig. 8: eine vergrößerte Ansicht des Schneidestempels gemäß der alternativen Ausführungsform der Biopsienadel;
- Fig. 9: eine Schnittansicht eines geteilten Kraftübertragungskolbens gemäß der alternativen Ausführungsform der Biopsienadel;
- Fig. 10: eine perspektivische Ansicht des Kraftübertragungskolbens einschließlich eines Klemmelements mit Hebel gemäß der alternativen Ausführungsform der Biopsienadel ohne Handteil;
- Fig. 11: eine perspektivische Ansicht des Kraftübertragungskolbens einschließlich des Klemmelements mit Hebel gemäß der alternativen Ausführungsform der Biopsienadel mit Handteil;
- Fig. 12: eine Schnittansicht des Handteils gemäß der alternativen Ausführungsform der Biopsienadel mit dem Klemmelement gemäß der alternativen Ausführungsform der Biopsienadel; und
- Fig. 13: eine Schnittansicht des hinteren Teils der alternativen Ausführungsform der Biopsienadel.

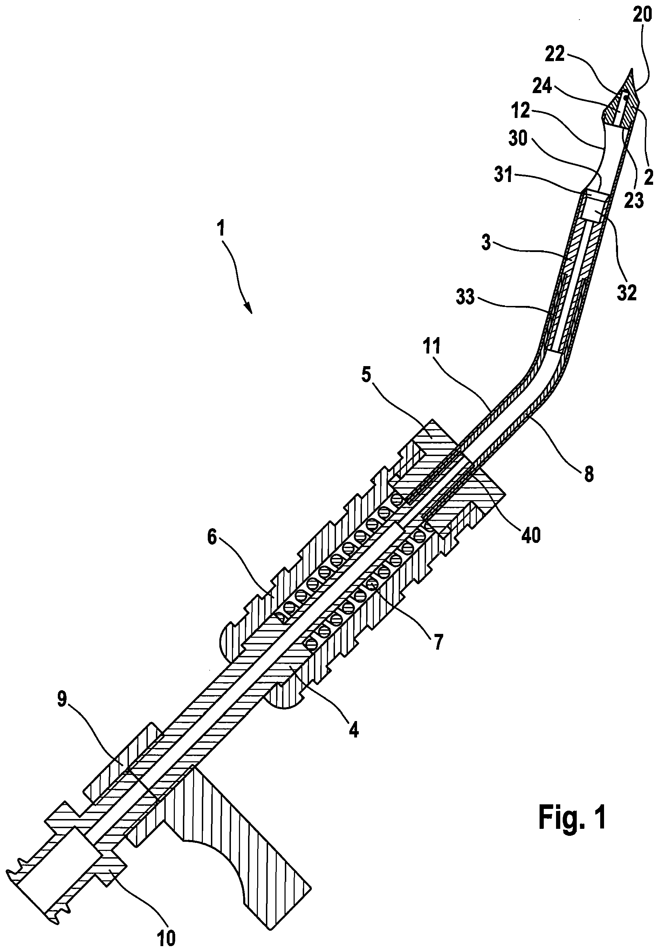

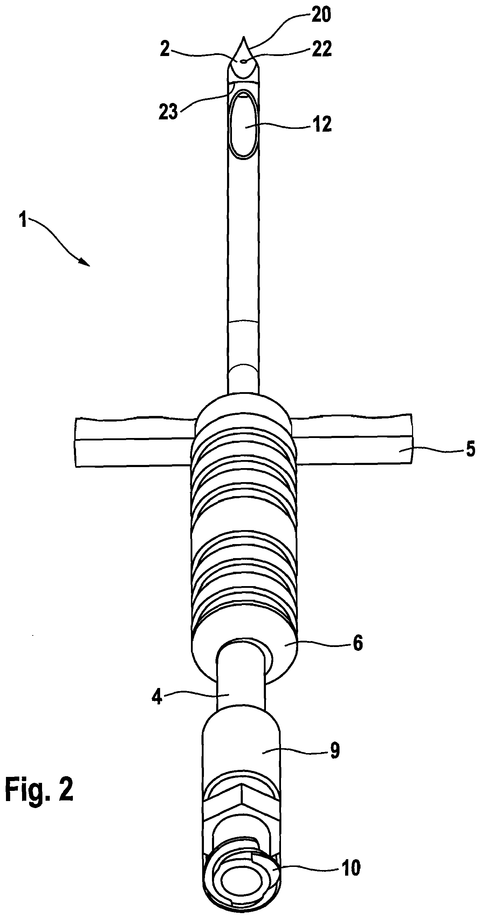

Die Figuren 1 und 2 zeigen eine Biopsienadel 1, wobei in Fig. 1 eine Schnittansicht der Biopsienadel 1 zu sehen ist. Hierbei ist eine Trokarspitze 2 der Biopsienadel 1 in Form einer mehrseitigen Schneidespitze mit konkaven Schneidekanten 20 ausgebildet.

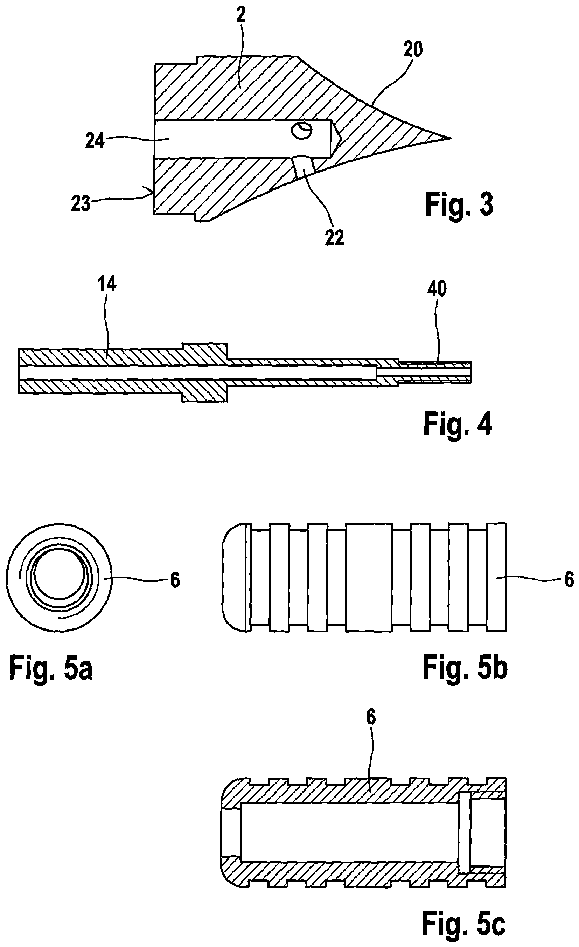

Wie in Fig. 3 vergrößert dargestellt, ist die Trokarspitze 2 mit einer dreiseitigen Schneidespitze ausgebildet, und weist auf jeder der drei Seiten eine Bohrung 22 auf, wobei sich diese Bohrungen 22 mittig in der Trokarspitze 2 treffen. Ferner weist die Trokarspitze 2 einen Kanal 24 zwischen dem Schnittpunkt der Bohrungen 22 und einer Grundfläche 23 der Trokarspitze 2 auf.

Fig. 1 zeigt weiter eine Führungshülse 8, die an der Grundfläche 23 der Trokarspitze 2 angrenzt, und in einem vorderen Bereich, nahe der Trokarspitze 2, eine seitliche Ausnehmung 12 aufweist, welche vorliegend oval ausgebildet ist. Die Führungshülse 8 ist aus einem im Wesentlichen steifen, biokompatiblen Material hergestellt.

Wie in Fig. 1 dargestellt, weist die Führungshülse 11 an einer Stelle zwischen der Trokarspitze 2 und einem Handteil 6 einen gekrümmten Verlauf auf. Winkel und Radius der Krümmung können beliebig, je nach Wunsch und Bedürfnis der behandelnden Person bzw. den Anforderungen der jeweiligen Behandlung, ausgebildet sein. In der vorliegenden Ausführungsform liegt der Winkel der Krümmung bei etwa 30°.

Die Biopsienadel 1 enthält zudem einen in Längsrichtung der Führungshülse 11 darin verschiebbaren Stempel 3, der auf einer vorderen Seite 30, in Richtung der Trokarspitze 2, eine Schneide 31 aufweist, wobei das im Bereich der seitlichen Ausnehmung 12 der Führungshülse 11 zu entnehmende Gewebe, das durch die seitliche Ausnehmung in die Führungshülse gelangen kann, durch die Schneide 31 abtrennbar ist. Die Schneide 31 ist hierbei über den gesamten Umfang der vorderen Seite 30 ausgebildet. Der Stempel 3 ist in seinem Querschnitt der Form und dem Verlauf der Führungshülse angepaßt.

Darüber hinaus weist der Stempel 3 in seinem Inneren einen zur Schneide 31 hin geöffneten Hohlraum 32 auf, in welchen das zu sammelnde Gewebe aufnehmbar ist. Form und Volumen der Ausnehmung können beliebig ausgebildet sein, wobei sich in der vorliegenden bevorzugten Ausführungsform eine zylindrische Bohrung in Verwendung und Herstellung als besonders praktikabel ergeben hat.

Ferner weist die Biopsienadel 1, wie in Fig. 1 dargestellt, ein in der Führungshülse 11 geführtes flexibles Kraftübertragungselement 8 auf, über welches der Stempel 3 in der Führungshülse 11 verschiebbar ist, wobei das Kraftübertragungselement 8 in der vorliegenden Ausführungsform aus einem flexiblen Kunststoffschlauch ausgebildet ist.

Die Führungshülse 11 ist vorliegend in einem Handteil 6 eingebracht. Die Kraft auf das Kraftübertragungselement 8 ist dabei über eine Betätigungsvorrichtung 9 und einen in dem Handteil 6 geführten Kraftübertragungskolben 4 übertragbar sein, wobei sich an einem dem Kraftübertragungskolben 4 zugewandten Ende des Stempels 3 eine erste Aufnahme 33 und an einem dem Stempel 3 zugewandten Ende des Kraftübertragungskolbens 4 eine zweite Aufnahme 40 für das Kraftübertragungselement 8 befinden.

Die erste Aufnahme 33 und die zweite Aufnahme 40 sind bevorzugt als Gewinde ausgebildet, auf welchen das obenstehend beschriebene Kraftübertragungselement 8 aufgeschraubt werden kann.

Wie in den Figuren 4, 5a, 5b und 5c gezeigt ist, sind ein distaler Schaft 14 des Kraftübertragungskolbens 4 und ein zugeordnetes Führungsloch im Handteil 6 jeweils exzentrisch ausgebildet. Somit kann der distale Schaft 14 des Kraftübertragungskolbens 4 gegenüber dem zugeordneten Führungsloch im Handteil 6 nicht mehr verdreht werden, wodurch die radiale Position immer dieselbe ist. Dadurch kann das Kraftübertragungselement 8 bzw. der Kunststoffschlauch, im Falle der vorliegenden Schraubverbindung mit der ersten und der zweiten Aufnahme 33, 40 nicht mehr durch Verdrehung in die falsche Richtung geöffnet werden.

In der Biopsienadel bzw. dem Handteil ist außerdem ein Rückstell-Federelement 7, welches vorliegend als Schraubenfeder ausgebildet ist, für den Stempel 3 angeordnet, welches den Stempel 3 nach Beendigung des Schneidevorgangs über das Kraftübertragungselement 8 und den Kraftübertragungskolben 4 in seine Grundposition zurückführt. In der vorliegenden Ausfiihrungsform wird die Vorspannung auf das Rückstell-Federelement 7 über einen Gegenhalter 5, welcher sich am proximalen Ende des Handteils 6 befindet, und den Kraftübertragungskolben 4, welcher am distalen Ende des Handteils ausgebildet ist, aufgebracht.

An dem der Trokarspitze 2 entgegengesetzten Ende der Biopsienadel 1, am distalen Ende des Handteils 6, ist ferner eine Anschlusseinrichtung 10 für eine Druckerzeugungsvorrichtung (nicht dargestellt) vorgesehen, mittels der ein Medikament, während sich die Biopsienadel 1 in dem zu untersuchenden Bereich befindet, über eine durchgehende Kanülierung durch das Handteil 6, das schlauchförmige Kraftübertragungselement 8, den Stempel 3, und die Trokarspitze 2 in den zu behandelnden Bereich injizierbar ist. Die Anschlusseinrichtung 10 ist vorliegend in Form eines Luer-Lock-Anschlusses ausgebildet, an welchem eine Spritze (nicht dargestellt) angebracht werden kann.

Nachfolgend wird ein Verfahren zur Entnahme von Gewebeproben im Detail beschrieben. Dabei wird die erfindungsgemäße Biopsienadel 1 zuerst in einen zu untersuchenden Bereich in einem Körper eingeführt, wobei sich die Trokarspitze 2 bis zum Gewebeentnahmebereich durch den Körper schneidet.

Die behandelnde Person greift die Biopsienadel dabei so, daß Mittelfinger und Zeigefinger auf dem Gegenhalter 5, und der Daumen auf der Betätigungsvorrichtung 9 aufgebracht werden. Beim Eindringen in den Körper kann die Betätigungsvorrichtung 9 je nach Ermessen der behandelnden Person niedergedrückt gehalten werden oder nicht.

Wenn die Biopsienadel 1 in die gewünschte Position eingeführt ist, wird der Stempel 3 über die Betätigungsvorrichtung 9, den Kraftübertragungskolben 4 und das Kraftübertragungselement 8 niedergedrückt, um im Bereich der seitlichen Ausnehmung 12 der Führungshülse 11 durch die Schneide 31 zu sammelndes Gewebe, das durch die seitliche Ausnehmung 12 in die Führungshülse 11 gelangen konnte, abzutrennen und in den Hohlraum 32 des Stempels 3 aufzunehmen.

Hierbei wird die Kraft, welche über die Betätigungsvorrichtung 9 auf den Kraftübertragungskolben 4 gebracht wird, auf das Kraftübertragungselement 8 übertragen, welches durch die Wandung der Führungshülse 11 begrenzt die resultierende Kraft auf den Stempel 3 überträgt. Dabei kann das Kraftübertragungselement 8 nicht nach Innen knicken, da die Querkontraktion immer auf die Wandung der Führungshülse 11 wirkt. Das Kraftübertragungselement 8 überträgt die Kraft somit um den gewünschten Bogen bzw. die vorgesehene(n) Krümmung(en) der Führungshülse 11.

Danach, wenn sich das zu entnehmende Gewebe im Hohlraum 32 befindet, wird die Biopsienadel aus dem zu untersuchenden Bereich herausgenommen, während sich der Stempel 3 formschlüssig auf der Grundfläche 23 der Trokarspitze befindet, d.h., während die Betätigungsvorrichtung 9 komplett niedergedrückt ist.

Abschließend, wenn die Biopsienadel aus dem Körper herausgenommen ist, kann die Betätigungsvorrichtung 9 gelöst bzw. losgelassen werden. Nun, nachdem sich der Stempel 3 wieder in seiner Grundposition befindet, kann die Gewebeprobe aus dem Hohlraum 32 entnommen werden.

Während sich die Biopsienadel 1 im Gewebeentnahmebereich oder einem anderen Bereich im Körper befindet, kann durch die Biopsienadel 1 ein Mittel, beispielsweise ein Medikament zur Behandlung des zu untersuchenden Bereichs, in den Körper injiziert werden. Hierfür kann eine Spritzen-/Kolbenvorrichtung an der Anschlusseinrichtung 10 angebracht werden, über welche das Mittel bzw. die Arznei durch den Kraftübertragungskolben 4, das Kraftübertragungselement 8, den Stempel 3 und die Trokarspitze 2 in den ausgewählten Bereich im Körper injiziert wird. Dies kann entweder bei niedergedrücktem Stempel 3 durch die Bohrung 22 (Kanal 24 und Bohrungen 22), oder bei nicht-niedergedrücktem Stempel 3 durch die seitliche Ausnehmung 12 erfolgen.

Die Erfindung läßt neben der erläuterten Ausführungsform weitere Gestaltungsansätze zu.

Das Handteil 6 ist, wie in den Figuren 1 und 5a bis 5c dargestellt, mit einer Strukturierung vorgesehen, welche in weiteren Ausführungsformen beliebig ausgestaltet sein kann. Auch glatte und/oder rutschfeste Oberflächen sind denkbar.

Anstelle der dreiseitigen Trokarspitze 2, kann diese eine beliebige andere Anzahl und Form von Schneideflächen bzw. -kanten aufweisen, und als konische, konkave oder konvexe Rundspitze ausgebildet sein. Hierbei kann die Trokarspitze 2 auf mindestens einer Seitenfläche 20 wenigstens eine Bohrung 22 aufweisen. Alternativ kann auch eine einzige durchgehende Bohrung bzw. beliebig geformte Öffnung, oder eine der Schneideflächenzahl entsprechende, sich ebenfalls mittig treffende Anzahl von Bohrungen bzw. Öffnungen durch die Trokarspitze 2 ausgebildet sein.

In der bevorzugten Ausfiihrungsform ist die Krümmung der Führungshülse 11 in einem Winkel von etwa 30° ausgebildet. Dieser kann für weitere Anwendungsfälle jedoch auch in einem Bereich zwischen 20° und 60°, in einer besonders bevorzugten Ausführungsform der Erfindung in einem Bereich zwischen 25° und 35° liegen. Es sind auch weitere, zusätzliche Krümmungen der Führungshülse 11 zu anderen Winkeln und/oder Radien denkbar.

Anstelle der im Wesentlichen steifen Führungshülse 11 ist auch eine Führungshülse aus einem flexiblen biokompatiblen Material denkbar.

Generell ist jedoch die im Wesentlichen steife Führungshülse 11 zu bevorzugen. Dabei kann die steife Führungshülse beispielsweise aus gehärtetem Stahl 1.4034 (gehärtet auf 52 bis 54 HRC) oder gehärtetem Stahl 1.4057 (gehärtet auf 52 bis 54 HRC) bestehen.

Durch eine steife bzw. starre Führungshülse kann trotz der bei einer Behandlung auftretenden Biege-, Schub- und/oder Torsionsbelastungen ein sicheres und genaues Vordringen in den Zielbereich gewährleistet werden. Allgemein können als Ausgangsmaterial für die Führungshülse auch sogenannte Messerstähle, INOX-Stähle oder VA-Stähle dienen, wobei es ein Ziel ist, ein Optimum aus guter Bioverträglichkeit, guter chemischer Beständigkeit, Rostfreiheit, hoher Härte, und martensitischem Gefüge vorzusehen.

Derlei für eine im Wesentlichen steife bzw. starre Führungshülse geeignete Stähle weisen beispielsweise eine Zugfestigkeit von 780 MPa bis 1.000 MPa, vorzugsweise von 800 MPa bis 980 MPa, besonders bevorzugt von 830 MPa bis 950 MPa auf. Geeignete Stähle zeigen beispielsweise eine Dehnung von 10% bis 14%, vorzugsweise um 12%.Der E-Modul geeigneter Stähle liegt beispielsweise in einem Bereich von 205 N/mm²x10³ bis 225 N/mm²x10³, vorzugsweise um 215 N/mm²x10³. Hinsichtlich der Streckgrenze geeigneter Stähle kann für die Streckgrenze ein beispielhafter Bereich von 550 bis 700 N/mm², bevorzugt von 575 bis 625 N/mm², besonders bevorzugt von 600 bis 650 N/mm² angegeben werden.

Im Rahmen von beispielhaften Materialprüfungen u.a. zur Untersuchung der Steifigkeit der Führungshülse haben sich hierbei zum Beispiel die folgenden zwei Stähle als besonders geeignet erwiesen:
1.4034:
   Biegefestigkeit / Zugfestigkeit: 850 MPa
   Dehnung: 12%
   E-Modul: 215 N/mm²x10³
   Streckgrenze: 600 N/mm²
1.4057:
   Biegefestigkeit / Zugfestigkeit: 930 MPa
   Dehnung: 12%
   E-Modul 215 N/mm²x10³
   Streckgrenze: 650 N/mm²

Diese vorstehend beispielhaft genannten Parameter können z.B. mit den aus der mechanischen Werkstoffprüfung bekannten Versuchen ermittelt werden. Beispielhaft sei hier auf den geläufigsten, klassischen Zugversuch verwiesen, der im Detail in der DIN EN 10002 (vormals DIN 50125, DIN 50145) beschrieben ist.

Das vorstehend beispielhaft fiir eine steife Führungshilfe vorgeschlagene Material kann bei der hier diskutierten erfindungsgemäßen Gelenk-Biopsienadel u.a. auch für die Trokarspitze und/oder den Stempel besonders gut verwendet werden. Die weiteren Bestandteile können alternativ aus ähnlichen Materialen oder beispielsweise auch aus Kunststoff bestehen.

Die Führungshülse 8 ist dabei nicht auf einen kreisförmigen Querschnitt beschränkt, sondern kann auch elliptisch, rechteckig oder in einer beliebigen anderen Querschnittsform ausgebildet sein.

Die Schneide des Stempels 3 kann in einer alternativen Ausführungsform nur über einen Teil der vorderen Seite 30 bzw. deren Rand, und/oder im inneren Bereich der vorderen Seite 30 des Stempels 3 ausgebildet sein.

In einer weiteren Ausführungsform kann das schlauchförmige, flexible Kraftübertragungselement 8 aus z.B. einem elliptischen oder mehreckigen Hohl- oder Massivprofil ausgebildet sein. Zudem kann es mit dem Stempel 3 und dem Kraftübertragungskolben 4 je nach Ausfiihrungsform des Kraftübertragungselements 8 und des Stempels 3 auch steckverbunden, verhakt, verklebt, verlötet und/oder verschweißt sein.

Falls keine oder nur eine geringe Krümmung der Führungshülse 11 vorliegt, oder das Kraftübertragungselement 8 nicht verschraubt, sondern wie obenstehend erwähnt, auf eine andere Art und Weise mit dem Stempel 3 und dem Kraftübertragungskolben 4 verbunden ist, kann auf die exzentrische Ausbildung des hinteren Schafts 14 des Kraftübertragungskolbens 4 und der Bohrung in dem Handteil 6 auch verzichtet werden, bzw. diese zentrisch ausgebildet sein.

Anstelle des in Fig. 1 dargestellten Rückstell-Federelements 7, kann in einer weiteren Ausführungsform auch ein beliebiges anderes Rückstellelement wie z.B. eine Luftfeder, eine Elastomerfeder, eine Evolutfeder, etc. in bzw. an dem Handteil 6 verspannt sein, welches den Stempel 3 wieder in seine Ursprungsposition zurückführen kann.

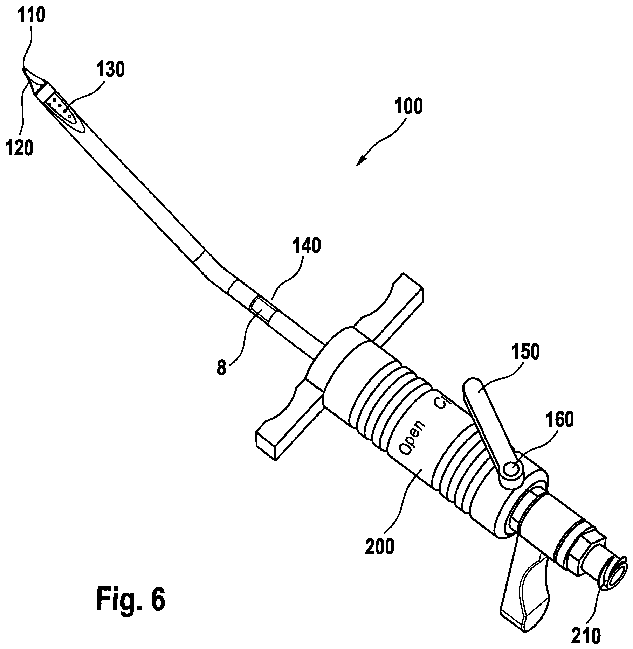

Fig. 6 zeigt eine perspektivische Gesamtansicht einer alternativen Ausführungsform der Biopsienadel. Bei einer solchen alternativen Ausfiihrungsform 100 ist in der Führungshülse ein Sichtfenster 140 auf das Kraftübertragungselement (8) integriert. Da das Kraftübertragungselement, welches beispielsweise ein PTFE-Schlauch ist, nahezu transparent ist, eine Gelenk- oder andere Körperflüssigkeit jedoch nicht, kann hierdurch schnell und einfach erkannt werden, ob sich die behandelnde Person mit der Biopsienadel 100 an der richtige Stelle befindet.

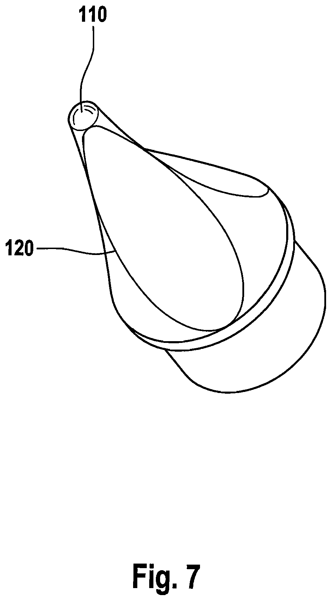

Fig. 6 und Fig. 7 zeigen weiter, dass die Trokarspitze in einer alternativen Ausfiihrungsform auch ohne Bohrungen 22 vorgesehen sein kann. Zudem können die Schneiden, wie in Fig. 7 dargestellt, abgerundet und die Spitze als Halbkugel ausgebildet sein. Dadurch kann der behandelnden Person bei einer Behandlung ein besseres Gefühl vermittelt werden, wo sich die Trokarspitze gerade im Gewebe befindet. Außerdem können hierdurch unerwünschte Verletzungen des Gewebes vermieden werden.

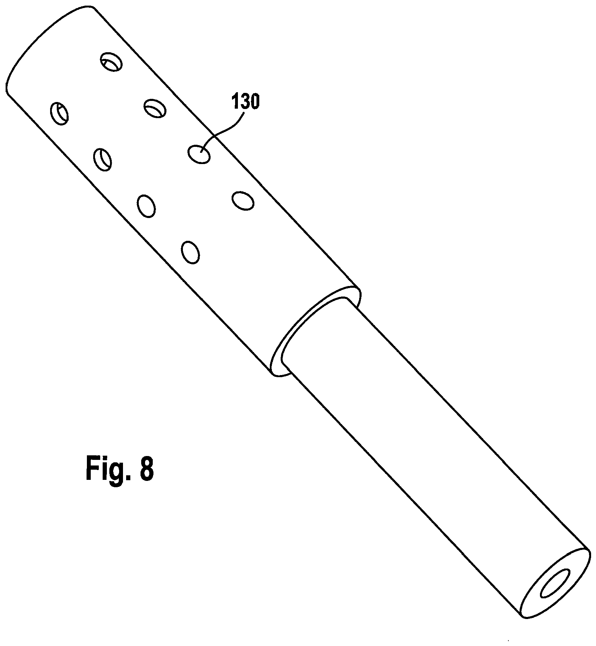

Die zum Flüssigkeitstransfer vorgesehenen Bohrungen, die bei der vorstehend diskutierten Beschreibung in der Trokarspitze vorgesehen waren, können, wie in Fig. 6 und Fig. 8 dargestellt, alternativ (oder zusätzlich) als Querbohrungen 130 im Schneidestempel integriert sein.

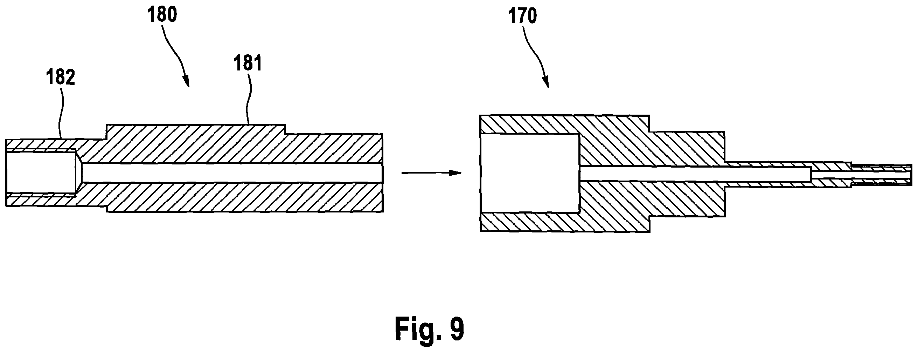

Ferner kann der Kraftübertragungskolben hinsichtlich herstellungstechnischer und wirtschaftlicher Aspekte nicht nur einteilig, sondern auch zwei- oder mehrteilig (170, 180) ausgestaltet sein. Die einzelnen Teile können dann einfach mit beispielsweise biokompatiblem Klebstoff verbunden werden, wobei alternativ natürlich auch Rast-, Steck-, Schweiß-, und/oder Lötverbindungen denkbar sind. Eine Ausführungsform einer mehrteiligen Ausgestaltung ist in Figur 9 gezeigt.

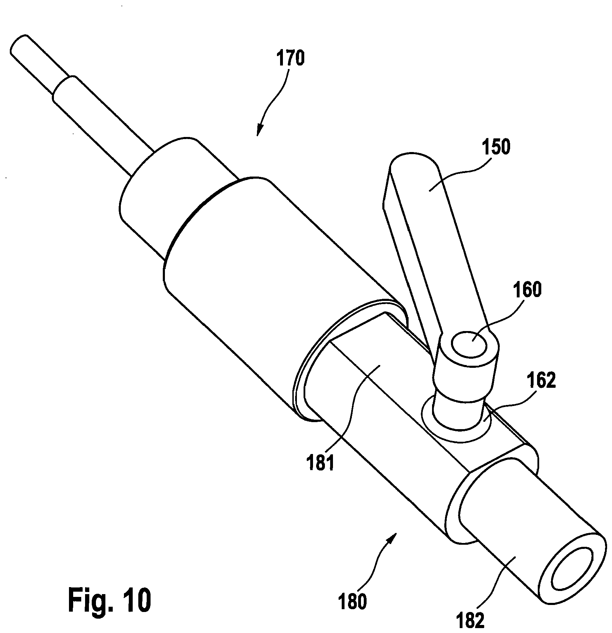

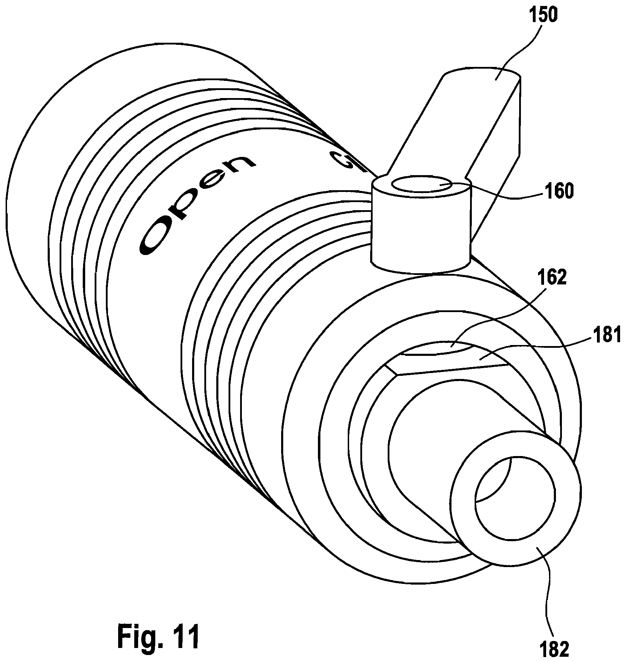

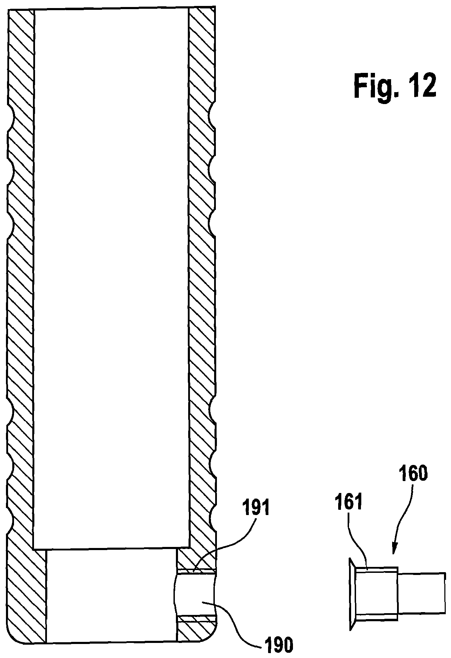

Wie in den Figuren 10 bis 12 dargestellt, kann die vorstehend diskutierte Verdrehsicherung durch die exzentrische Anordnung des distalen Schafts des Kraftübertragungskolbens und des zugeordneten Führungslochs in einem Handteil 200, die auch zur Positionsfixierung dienen kann, alternativ auch über einen Hebel 150 geschaffen werden, der ein Klemmelement 160 über ein Gewinde 161 auf eine Fläche 181 eines Teils des Kraftübertragungskolbens drückt. Damit kann eine Klemmung erzielt werden, durch welche der Kraftübertragungskolben 170, 180 ebenfalls in einer definierten Position gehalten werden kann. Dafür ist im Handteil 200 wie in Fig. 12 dargestellt eine Querbohrung 190 mit Gewinde 191 angebracht. Das Klemmelement kann hierbei so konstruiert sein, dass es von innerhalb des Handteils 200 durch die Gewindebohrung 191 geschraubt werden kann. Dies hat den Vorteil, dass auch ein Anschlag nach oben gewährleistet ist. Anschließend kann der Hebel 150 aufgeklebt oder aufgeschweißt, etc. werden. Um der behandelnden Person die Anwendung der vorliegenden Biopsienadel 100 zu erleichtern, kann auf der Außenseite der Biopsienadel 100 wie in Fig. 6 dargestellt die Öffnungs- und Verschlussrichtung des Hebels angezeigt sein. Ferner muss der Klemmmechanismus nicht wie vorstehend beschrieben ausgebildet sein. Das Klemmelement und der Hebel können beispielsweise auch aus einer Einheit gefertigt sein. Auch die Gewinde 161, 191 können durch ein alternatives Kraftübertragungssystem ersetzt werden.

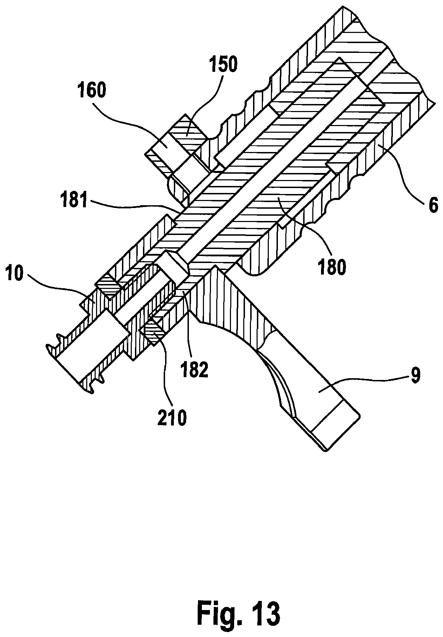

Wie in Fig. 13 dargestellt, kann die Betätigungsvorrichtung 9 frei drehbar auf dem Kraftübertragungskolben gelagert sein. Die Betätigungsvorrichtung 9, welche sich beispielsweise auf einem distalen Zapfen 182 des Kraftübertragungskolbens 4, 170, 180 befindet, kann hierbei durch eine Gegenscheibe 210 und die über ein Gewinde am Kraftübertragungskolben 4, 170, 180 anbringbare Anschlusseinrichtung 10 verblockt sein. Durch eine derartige Lagerung, mittels welcher die Betätigungsvorrichtung 9 in eine beliebige radiale Position gedreht werden kann, also relativ zum Handteil 6 bzw. zur Führungshülse 11 verdrehbar ist, steht es der behandelnden Person frei, mit welcher Hand sie die Gelenk-Biopsienadel bedient. Zudem ist die Gelenk-Biopsienadel somit für Links- und Rechtshänder gleichermaßen geeignet.

In alternativen Ausführungsformen der vorliegenden Biopsienadel können Gewinde durch glatte zylindrische Flächen ersetzt werden, wodurch Verbindungsteile geklebt oder geschweißt werden können, was den Vorteil einer wirtschaftlicheren Fertigung mit sich bringen kann.

Eine behandelnde Person kann mit einer Biopsienadel 100 nach den Figuren 6 bis 12, die sich im geschlossenen Zustand befindet, d.h. mit niedergedrücktem Schneidestempel 130, in einen Körper vordringen, bis eine Flüssigkeit am Sichtfenster 140 zu sehen ist. Sobald die Flüssigkeit das Sichtfenster 140 erreicht, öffnet die behandelnde Person die Biopsienadel 100, d.h., löst den von ihr ausgeübten Druck auf den Schneidestempel 130, und setzt an einem Lueranschluss 210 eine Spritze auf um einen Unterdruck zu erzeugen. Anschließend kann wie bereits vorstehend beschrieben verfahren werden.

Obwohl bestimmte Eigenschaften und Merkmale der vorliegenden Biopsienadel in den beigefügten Figuren auf gewisse Ausführungsbeispiele beschränkt sind, ist es als selbstverständlich zu betrachten, dass diese in weiteren, nicht diskutierten Ausführungsformen, in beliebiger Kombination Anwendung finden können.

Die vorstehend diskutierte Erfindung schafft damit eine Biopsienadel, insbesondere eine Gelenk-Biopsienadel, mit einer Trokarspitze und einer Führungshülse, die an eine Grundfläche der Trokarspitze angrenzt, und die in einem der Trokarspitze benachbarten Bereich eine seitliche Ausnehmung aufweist. Ferner weist diese Biopsienadeln einen in Längsrichtung der Führungshülse darin verschiebbaren Stempel auf, der auf einer auf die Trokarspitze zuweisenden Seite eine Schneide aufweist, wobei das im Bereich der seitlichen Ausnehmung der Führungshülse zu entnehmende Gewebe, das durch die seitliche Ausnehmung in die Führungshülse gelangen kann, durch die Schneide abtrennbar ist. Die Führungshülse ist dabei in einem Handteil festgelegt. Die Führungshülse weist hierbei erstmals an wenigstens einer Stelle zwischen der Trokarspitze und dem Handteil einen gekrümmten Verlauf auf, wobei der Stempel über ein in der Führungshülse geführtes, flexibles Kraftübertragungselement verschiebbar ist.

## Patentansprüche

1. Gelenk-Biopsienadel (1) zur Entnahme von Gewebeproben mit
einer Trokarspitze (2),
einer im Wesentlichen steifen Führungshülse (11), die an eine Grundfläche (23) der Trokarspitze (2) angrenzt, und in einem der Trokarspitze (2) benachbarten Bereich eine seitliche Ausnehmung (12) aufweist,
einem in Längsrichtung der Führungshülse (11) darin verschiebbaren Stempel (3), der auf einer auf die Trokarspitze (2) zuweisenden Seite (30) eine Schneide (31) aufweist,
wobei das im Bereich der seitlichen Ausnehmung (12) der Führungshülse (11) zu entnehmende Gewebe, das durch die seitliche Ausnehmung (12) in die Führungshülse (11) gelangen kann, durch die Schneide (3) abtrennbar ist, und
einem Handteil (6), in welchem die Führungshülse (11) festgelegt ist,
**dadurch gekennzeichnet, daß** die Führungshülse (11) an wenigstens einer Stelle zwischen der Trokarspitze (2) und dem Handteil (6) einen gekrümmten Verlauf aufweist,
der Stempel (3) über ein in der Führungshülse (11) geführtes, flexibles Kraftübertragungselement (8) verschiebbar ist,
eine Kraft auf das flexible Kraftübertragungselement (8) über eine Betätigungsvorrichtung (9) und einen in dem Handteil (6) geführten Kraftübertragungskolben (4) übertragbar ist,
sich an einem dem Kraftübertragungskolben (4) zugewandten Ende des Stempels (3) eine erste Aufnahme (33) und an einem dem Stempel (3) zugewandten Ende des Kraftübertragungskolbens (4) eine zweite Aufnahme (40) für das Kraftübertragungselement (8) befinden,
ein distaler Schaft (14) des Kraftübertragungskolbens (4) und ein zugeordnetes Führungsloch im Handteil (6) verdrehsicher ausgebildet sind, und
der distale Schaft (14) des Kraftübertragungskolbens (4) und das zugeordnete Führungsloch im Handteil (6) jeweils exzentrisch und komplementär zueinander ausgebildet sind.

2. Gelenk-Biopsienadel nach Anspruch 1, **dadurch gekennzeichnet, daß** das flexible Kraftübertragungselement (8) ein Kunststoffschlauch ist.

3. Gelenk-Biopsienadel nach Anspruch 1 oder 2, **dadurch gekennzeichnet, daß** der Stempel (3) im Inneren einen zur Schneide (31) hin geöffneten Hohlraum (32) aufweist, in welchen das zu sammelnde Gewebe aufnehmbar ist.

4. Gelenk-Biopsienadel nach einem der Ansprüche 1 bis 3, **dadurch gekennzeichnet, daß** die Trokarspitze (2) in Form einer mehrseitigen Schneidespitze mit konkaven Schneidekanten (20) ausgebildet ist.

5. Gelenk-Biopsienadel nach einem der Ansprüche 1 bis 4, **dadurch gekennzeichnet, daß** sich auf mindestens einer Seite der Trokarspitze (2) wenigstens eine Bohrung (22) befindet, welche einen Durchfluß einer Körperflüssigkeit durch die Trokarspitze (2) erlaubt.

6. Gelenk-Biopsienadel nach einem der Ansprüche 1 bis 5, **dadurch gekennzeichnet, daß** am distalen Ende des Handteils (6) eine Anschlusseinrichtung (10) für eine Druckerzeugungsvorrichtung ausgebildet ist, mittels der ein Medikament über eine durchgehende Kanülierung durch das Handteil (6), das Kraftübertragungselement (8), den Stempel (3), und die Trokarspitze (2) in den zu behandelnden Bereich injizierbar ist.

7. Gelenk-Biopsienadel nach einem der Ansprüche 1 bis 6, **dadurch gekennzeichnet, daß** der Kraftübertragungskolben (4, 170, 180), durch den eine Kraft auf das flexible Kraftübertragungselement (8) übertragbar ist, vorzugsweise mehrteilig ausgebildet ist und in einer definierten Position gehalten werden kann.

8. Gelenk-Biopsienadel nach einem der Ansprüche 1 bis 7, **dadurch gekennzeichnet, daß** der Kraftübertragungskolben (4, 170, 180) mittels einer Kraftübertragungseinrichtung (150), die ein Klemmelement (160) auf eine Fläche (181) des Kraftübertragungskolbens (4, 170, 180) drückt, in einer definierten Position gehalten werden kann, wobei die Kraftübertragungseinrichtung (150) vorzugsweise ein Hebel ist, der das Klemmelement (160) über ein Gewinde (161, 191) auf die Fläche (181) des Kraftübertragungskolbens (4, 170, 180) drückt.

9. Gelenk-Biopsienadel nach einem der Ansprüche 1 bis 8, **dadurch gekennzeichnet, daß** im Handteil (6) ein Rückstell-Federelement (7) für den Stempel (3) angeordnet ist.

10. Gelenk-Biopsienadel nach einem der Ansprüche 1 bis 9, **dadurch gekennzeichnet, daß** in der Führungshülse (11) ein Sichtfenster (140) integriert ist, das eine direkte Sicht auf das Kraftübertragungselement (8) erlaubt.

11. Gelenk-Biopsienadel nach einem der Ansprüche 1 bis 10, **dadurch gekennzeichnet, daß** die Spitze (110) der Trokarspitze als Halbkugel ausgebildet ist.

12. Gelenk-Biopsienadel nach einem der Ansprüche 1 bis 3 und 5 bis 11, **dadurch gekennzeichnet, daß** die Schneiden (120) der Trokarspitze abgerundet sind.

13. Gelenk-Biopsienadel nach einem der Ansprüche 1 bis 12, **dadurch gekennzeichnet, daß** Querbohrungen (130) im Stempel (3) integriert sind.

14. Gelenk-Biopsienadel nach einem der Ansprüche 1 bis 13, **dadurch gekennzeichnet, daß** die Betätigungsvorrichtung (9) um die Längsachse der Gelenk-Biopsienadel frei drehbar auf dem Kraftübertragungskolben (4, 170, 180) angeordnet ist.

## Claims

1. A joint biopsy needle (1) for taking tissue samples, comprising
a trocar tip (2),
a substantially rigid guide sleeve (11) which adjoins a base surface (23) of the trocar tip (2) and which has a lateral opening (12) in a region adjacent to the trocar tip (2),
a plunger (3) which can be displaced in the guide sleeve (11) in the longitudinal direction thereof and has a cutting blade (31) on a side (30) that faces the trocar tip (2),
wherein the tissue which is to be taken in the region of the lateral opening (12) of the guide sleeve (11) and can enter the guide sleeve (11) through the lateral opening (12) can be severed by the cutting blade (3), and
a handle (6) in which the guide sleeve (11) is immobilized,
**characterized in that** the guide sleeve (11) is curved at least in one location between the trocar tip (2) and the handle (6),
the plunger (3) can be displaced by means of a flexible force transmitting element (8) that is guided in the guide sleeve (11),
a force may be transmitted to the flexible force transmitting element (8) via an actuating device (9) and a force transmitting piston (4) which is guided in the handle (6),
a first reception (33) is located at an end of the plunger (3) facing the force transmitting piston (4), and a second reception (40) for the force transmitting element (8) is located at an end of the force transmitting piston (4) facing the plunger (3),
a distal shaft (14) of the force transmitting piston (4) and an associated guide hole in the handle (6) are realized so as to be secured against rotation, and
the distal shaft (14) of the force transmitting piston (4) and the associated guide hole in the handle (6) are each realized to be eccentric and complementary relative to each other.

2. The joint biopsy needle according to claim 1, **characterized in that** the flexible force transmitting element (8) is a plastic tube.

3. The joint biopsy needle according to claim 1 or 2, **characterized in that** the plunger (3) has on its inside a hollow space (32) which is open toward the cutting blade (31) and in which the tissue to be collected may be received.

4. The joint biopsy needle according to any one of claims 1 to 3, **characterized in that** the trocar tip (2) is realized in the form of a cutting tip having a plurality of sides with concave cutting blades (20).

5. The joint biopsy needle according to any one of claims 1 to 4, **characterized in that** on at least one side of the trocar tip (2) there is at least one bore (22) which allows a body fluid to flow through the trocar tip (2).

6. The joint biopsy needle according to any one of claims 1 to 5, **characterized in that** on the distal end of the handle (6) a coupling means (10) for a pressure generating device is formed, by means of which a drug may be injected into the region to be treated via a continuous cannulation through the handle (6), the force transmitting element (8), the plunger (3), and the trocar tip (2).

7. The joint biopsy needle according to any one of claims 1 to 6, **characterized in that** the force transmitting piston (4, 170, 180) whereby a force may be transmitted to the flexible force transmitting element (8) may preferably be realized in the form of several parts (170, 180) and may be held in a defined position.

8. The joint biopsy needle according to any one of claims 1 to 7, **characterized in that** the force transmitting piston (4, 170, 180) may be held in a defined position by means of a force transmitting means (150) which urges a clamping member (160) against a surface (181) of the force transmitting piston (4, 170, 180), the force transmitting means (150) preferably being a lever which urges the clamping member (160) via a thread (161) against the surface (181) of the force transmitting piston (4, 170, 180).

9. The joint biopsy needle according to any one of claims 1 to 8, **characterized in that** a reset spring member (7) for the plunger (3) is arranged inside the handle (6).

10. The joint biopsy needle according to any one of claims 1 to 9, **characterized in that** a window (140) allowing direct viewing of the force transmitting member (8) is integrated in the guide sleeve (11).

11. The joint biopsy needle according to any one of claims 1 to 10, **characterized in that** the tip (110) of the trocar tip is realized to be hemispherical.

12. The joint biopsy needle according to any one of claims 1 to 3 and 5 to 11, **characterized in that** the cutting blades (120) of the trocar tip are rounded.

13. The joint biopsy needle according to any one of claims 1 to 12, **characterized in that** transverse bores (130) are integrated in the plunger (3).

14. The joint biopsy needle according to any one of claims 1 to 13, **characterized in that** the actuating device (9) is arranged on the force transmitting piston (4, 170, 180) so as to be freely rotatable about the longitudinal axis of the joint biopsy needle.

## Revendications

1. Aiguille de biopsie articulée (1) pour le prélèvement d'échantillons tissulaires avec
une pointe de trocart (2),
une gaine de guidage (11) sensiblement rigide, qui jouxte une surface de base (23) de la pointe de trocart (2), et présente un évidement latéral (12) dans une zone adjacente à la pointe de trocart (2),
un poinçon (3) qui peut être déplacé dans le sens longitudinal de la gaine de guidage (11) et qui présente une lame (31) sur un côté (30) tourné vers la pointe de trocart (2),
dans laquelle le tissu qui est à prélever dans la zone de l'évidement latéral (12) de la gaine de guidage (11) et qui peut arriver par l'évidement latéral (12) jusque dans la gaine de guidage (11), peut être excisé par la lame (3), et
une pièce à main (6), dans laquelle la gaine de guidage (11) est fixée,
**caractérisée en ce que** la gaine de guidage (11) présente un profil incurvé à au moins un endroit entre la pointe de trocart (2) et la pièce à main (6),
le poinçon (3) peut être déplacé par le biais d'un élément de transmission de force (8) flexible, guidé dans la gaine de guidage (11),
une force peut être transmise sur l'élément de transmission de force (8) flexible par le biais d'un dispositif d'actionnement (9) et d'un piston de transmission de force (4) guidé dans la pièce à main (6),
pour l'élément de transmission de force (8), un premier logement (33) se trouve au niveau d'une extrémité du poinçon (3) tournée vers le piston de transmission de force (4) et un deuxième logement (40) se trouve au niveau d'une extrémité du piston de transmission de force (4) tournée vers le poinçon (3),
un arbre distal (14) du piston de transmission de force (4) et un trou de guidage associé dans la pièce à main (6) sont réalisés résistants à la torsion, et
l'arbre distal (14) du piston de transmission de force (4) et le trou de guidage associé dans la pièce à main (6) sont réalisés respectivement de manière excentrique et complémentaire l'un par rapport à l'autre.

2. Aiguille de biopsie articulée selon la revendication 1, **caractérisée en ce que** l'élément de transmission de force (8) flexible est un tuyau en plastique.

3. Aiguille de biopsie articulée selon la revendication 1 ou 2, **caractérisée en ce que** le poinçon (3) présente à l'intérieur une cavité (32) ouverte vers la lame (31), dans laquelle le tissu à collecter peut être reçu.

4. Aiguille de biopsie articulée selon l'une quelconque des revendications 1 à 3, **caractérisée en ce que** la pointe de trocart (2) est réalisée sous la forme d'une pointe de coupe multilatérale avec des arêtes de coupe concaves (20).

5. Aiguille de biopsie articulée selon l'une quelconque des revendications 1 à 4, **caractérisée en ce qu'**au moins un perçage (22), lequel permet un écoulement d'un fluide corporel à travers la pointe de trocart (2), se trouve sur au moins un coté de la pointe de trocart (2).

6. Aiguille de biopsie articulée selon l'une quelconque des revendications 1 à 5, **caractérisée en ce qu'**un dispositif de raccordement (10) pour un dispositif de génération de pression est réalisé au niveau de l'extrémité distale de la pièce à main (6), au moyen duquel un médicament peut être injecté par le biais d'une canule continue à travers la pièce à main (6), l'élément de transmission de force (8), le poinçon (3) et la pointe de trocart (2), dans la zone à traiter.

7. Aiguille de biopsie articulée selon l'une quelconque des revendications 1 à 6, **caractérisée en ce que** le piston de transmission de force (4, 170, 180), par lequel une force peut être transmise sur l'élément de transmission de force (8) flexible, est réalisé de préférence en plusieurs parties et peut être maintenu dans une position définie.

8. Aiguille de biopsie articulée selon l'une quelconque des revendications 1 à 7, **caractérisée en ce que** le piston de transmission de force (4, 170, 180) est maintenu dans une position définie au moyen d'un dispositif de transmission de force (150), qui pousse un élément de serrage (160) sur une surface (181) du piston de transmission de force (4, 170, 180), dans laquelle le dispositif de transmission de force (150) est de préférence un levier, qui pousse l'élément de serrage (160) sur la surface (181) du piston de transmission de force (4, 170, 180) par le biais d'un filetage (161, 191).

9. Aiguille de biopsie articulée selon l'une quelconque des revendications 1 à 8, **caractérisée en ce qu'**un élément de ressort de rappel (7) pour le poinçon (3) est agencé dans la pièce à main (6).

10. Aiguille de biopsie articulée selon l'une quelconque des revendications 1 à 9, **caractérisée en ce qu'**une fenêtre d'observation (140), qui permet une vue directe sur l'élément de transmission de force (8), est intégrée dans la gaine de guidage (11).

11. Aiguille de biopsie articulée selon l'une quelconque des revendications 1 à 10, **caractérisée en ce que** la pointe (110) de la pointe de trocart est réalisée en tant que demi-boule.

12. Aiguille de biopsie articulée selon l'une quelconque des revendications 1 à 3 et 5 à 11, **caractérisée en ce que** les lames (120) de la pointe de trocart sont arrondies.

13. Aiguille de biopsie articulée selon l'une quelconque des revendications 1 à 12, **caractérisée en ce que** des perçages transversaux (130) sont intégrés dans le poinçon(3).

14. Aiguille de biopsie articulée selon l'une quelconque des revendications 1 à 13, **caractérisée en ce que** le dispositif d'actionnement (9) est agencé sur le piston de transmission de force (4, 170, 180) de manière librement rotative autour de l'axe longitudinal de l'aiguille de biopsie articulée.
